# EUROPÄISCHE PATENTSCHRIFT

(11) **EP 2 672 846 B1**
(45) Veröffentlichungstag und Bekanntmachung des Hinweises auf die Patenterteilung: **17.08.2016**
(21) Anmeldenummer: 12708093.5
(22) Anmeldetag: 06.02.2012
(51) Int. Cl.: A24F 47/00, A61M 15/06, A61M 15/00

(54) **INHALATIONSVORRICHTUNG**
INHALATION DEVICE
DISPOSITIF D'INHALATION

(30) Priorität: 07.02.2011 DE 102011010532
(43) Veröffentlichungstag der Anmeldung: 18.12.2013
(73) Patentinhaber: S.A.S.C. AG, 6340 Baar (CH)
(72) Erfinder: BURGHARDT, Thorsten, 44581 Castrop-Rauxel (DE)
(74) Vertreter: Patentanwälte Dörner & Kötter PartG mbB
(86) Internationale Anmeldenummer: PCT/EP2012/051981
(87) Internationale Veröffentlichungsnummer: WO 2012/107414

(56) Entgegenhaltungen:
- WO-A2-2006/002445
- GB-A- 409 650
- US-A- 6 098 632
- US-A1- 2005 016 550
- US-A1- 2006 130 857

## Beschreibung

Die Erfindung betrifft eine Vorrichtung zum Inhalieren gasförmiger Medien, insbesondere Nikotin, die aus einem hülsenförmigen Körper besteht, der ein Depot aufnimmt, welches gasdicht verschlossen ist, und die ein in Ansaugrichtung dem Depot nachgeordnetes Mundstück sowie eine Aktivierungseinrichtung zum Öffnen des Depots aufweist.

Für das Inhalieren von gasförmigen Medien besteht eine Vielzahl von Anwendungsfällen. Insbesondere im medizinischen und therapeutischen Bereich findet die Inhalation von Gasen vielfältige Anwendung. Aber auch das Rauchen zählt zu den Anwendungsfällen, bei denen ein gasförmiges Medium inhaliert wird. Dabei bestehen handelsübliche Zigaretten meist aus in Papier oder einem Tabakblatt gewickeltem Tabak sowie einem am Mundstück angebrachten Filterelement. Durch Anzünden des vorderen Endes der Zigarette verglüht bzw. verschwelt der Tabak und setzt Nikotin frei, welcher durch den vom Raucher zu inhalierenden Rauch mitgerissen wird. Bei der Verbrennung bzw. Verschwelung des Tabaks werden neben Nikotin auch andere Substanzen freigesetzt bzw. erzeugt, die häufig gesundheitsschädlich sind, so Teer, Arsen- und cadmiumhaltige Verbindungen sowie kanzerogene Verbindung wie Hydrazin, Chrysen, Formaldehyd, Nietrosamine und ähnliches.

Seit geraumer Zeit wird versucht, so genannte rauchfreie Zigaretten auf den Markt zu etablieren. Hierbei handelt es sich um Produkte, bei denen der Tabak nur erwärmt wird, ohne verbrannt zu werden, so dass das gewünschte Nikotin freigesetzt werden soll, die Entstehung gesundheitsschädlicher Stoffe jedoch vermieden wird. Ein weiterer Ansatz für eine rauchfreie Zigarette ist in der DE 103 21 379 A1 geschildert. Bei dieser rauchfreien Zigarette wird mit Hilfe einer elektrischen Heizeinrichtung Luft erwärmt und die erwärmte Luft durch ein Einweg-Depot geleitet, in dem eine vorgegebene Menge Nikotin aufgenommen ist. Das Nikotin soll durch die erwärmte Luft freigesetzt und von dem Benutzer inhaliert werden.

Da die Unterbringung einer elektrischen Heizeinrichtung in der rauchfreien Zigarette aufwendig ist, ist eine rauchfreie Zigarette entwickelt worden, die ein Einweg-Depot enthält, in dem eine Trägersubstanz für das Nikotin vorgesehen ist, welche bei Umgebungstemperatur das Nikotin freisetzt (vgl. WO 2007/090594 A1). Das Depot ist dabei gasdicht verschlossen und wird bei Bedarf mit Hilfe einer Aktivierungseinrichtung geöffnet, sodass das in dem Depot vorgesehene Gas inhaliert werden kann. Außerdem sind aus der US 6 098 632 A ein nikotin-undurchdringbarer Behälter sowie ein Verfahren zu seiner Herstellung bekannt.

Die bekannte rauchfreie Zigarette erfüllt die an sie gestellten Anforderungen. Allerdings ist ihr Aufbau sehr komplex und daher die Handhabung umständlich. Zudem ist nachteilig, dass ein Nachfüllen nach dem Gebrauch nur sehr bedingt möglich ist.

Hier will die Erfindung Abhilfe schaffen. Der Erfindung liegt die Aufgabe zugrunde, eine Vorrichtung zum Inhalieren gasförmiger Medien, insbesondere Nikotin, zu schaffen, bei der mit weniger Bauteilen eine zuverlässige Funktion gewährleistet ist und die mehrfach verwendbar ist. Gemäß der Erfindung wird diese Aufgabe durch die Merkmale des Anspruchs 1 gelöst.

Mit der Erfindung ist eine Vorrichtung zum Inhalieren gasförmiger Medien, insbesondere Nikotin, geschaffen, die mit weniger Bauteilen eine zuverlässige Funktion gewährleistet und die mehrfach verwendbar ist. Zudem beinhaltet die Vorrichtung die aus dem Stand der Technik bekannten Vorteile des gesunden Rauchens. Die Verwendung einer Folie als Aktivierungseinrichtung bietet den Vorteil, einer platzsparenden und preiswerten Ausbildung. Die Verwendung einer selbstklebenden Folie führt zudem bei der Herstellung der rauchfreien Zigarette beziehungsweise der Depotträger zu Vorteilen bei der Handhabung.

In dem Körper ist vorzugsweise ein Depotträger angeordnet, der herausnehmbar ist. Der Depotträger ist in der Lage, das mit dem gasförmigen Medium befüllte Depot aufzunehmen. Aufgrund der Herausnehmbarkeit ist ein Austauschen des Depots einfach möglich, was das Nachfüllen erleichtert.

Eine besondere Ausführung besteht darin, in dem Körper eine Aufnahme auszubilden. Die Aufnahme kann das Depot unmittelbar aufnehmen, was den Aufbau der Vorrichtung vereinfacht. Alternativ kann die Aufnahme den Depotträger aufnehmen, der die Handhabung der Vorrichtung erleichtert.

In anderer Weiterbildung der Erfindung umfasst die Aktivierungseinrichtung eine Abziehvorrichtung. Mit Hilfe der Abziehvorrichtung ist eine Aktivierung der Zigarette und damit die Handhabung vor dem Genuss der Zigarette einfach und sehr bequem möglich.

Andere Weiterbildungen und Ausgestaltungen der Erfindung sind in den übrigen Unteransprüchen angegeben. Ein Ausführungsbeispiel der Erfindung ist in der Zeichnung dargestellt und wird nachfolgend im Einzelnen beschrieben. Es zeigen:
- Fig. 1: die perspektivische Darstellung einer rauchfreien Zigarette in einer ersten Ausbildung;
- Fig. 2: die explosionsartige Darstellung der in Figur 1 darstellten rauchfreien Zigarette;
- Fig. 3: verschiedene Schnittdarstellungen der in Figur 1 darstellten rauchfreien Zigarette;
- Fig. 4: die perspektivische Darstellung einer rauchfreien Zigarette in einer zweiten Ausbildung in nicht gebrauchsfertiger Position;
- Fig. 5: die perspektivische Darstellung der in Figur 4 dargestellten rauchfreien Zigarette in gebrauchsfertiger Position;
- Fig. 6: die explosionsartige Darstellung der in Figur 4 dargestellten rauchfreien Zigarette;
- Fig. 7: verschiedene Schnittdarstellungen der in Figur 4 dargestellten rauchfreien Zigarette;
- Fig. 8: die perspektivische Darstellung einer rauchfreien Zigarette in einer dritten Ausbildung mit abgeklapptem Griffstück;
- Fig. 9: die in Figur 8 dargestellte rauchfreie Zigarette mit Griffstück in Betätigungsposition;
- Fig. 10: die explosionsartige Darstellung der in Figur 8 dargestellten rauchfreien Zigarette;
- Fig. 11: verschiedene Schnittdarstellungen der in Figur 8 dargestellten rauchfreien Zigarette;
- Fig. 12: die perspektivische Darstellung des hülsenförmigen Körpers des Ausführungsbeispiels nach Figur 8;
- Fig. 13: die perspektivische Darstellung einer rauchfreien Zigarette in einer vierten Ausbildung;
- Fig. 14: die explosionsartige Darstellung der in Figur 13 dargestellten rauchfreien Zigarette;
- Fig. 15: verschiedene Schnittdarstellungen der in Figur 13 dargestellten rauchfreien Zigarette;
- Fig. 16: die perspektivische Darstellung einer rauchfreien Zigarette in einer fünften Ausbildung;
- Fig. 17: die explosionsartige Darstellung der in Figur 16 dargestellten rauchfreien Zigarette;
- Fig. 18: die perspektivische Darstellung der in Figur 16 dargestellten rauchfreien Zigarette in gebrauchsfertiger Position.

Die als Ausführungsbeispiel gewählte Vorrichtung zum Inhalieren gasförmiger Medien, insbesondere Nikotin, besteht aus einem hülsenförmigen Körper 1. In dem Körper 1 ist ein Depot 3 angeordnet. Die Vorrichtung weist ein dem Depot 3 in Ansaugrichtung nachgeordnetes Mundstück 4 auf sowie eine Aktivierungseinrichtung 5 zum Öffnen des Depots 3. Die Vorrichtung ist nachfüllbar.

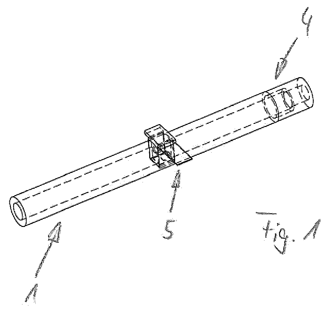

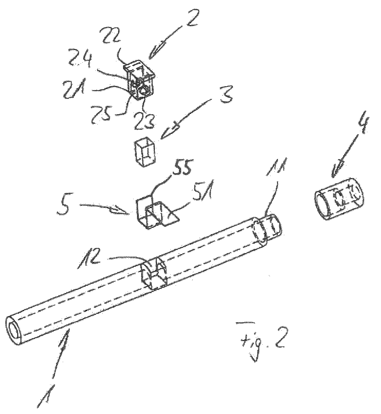

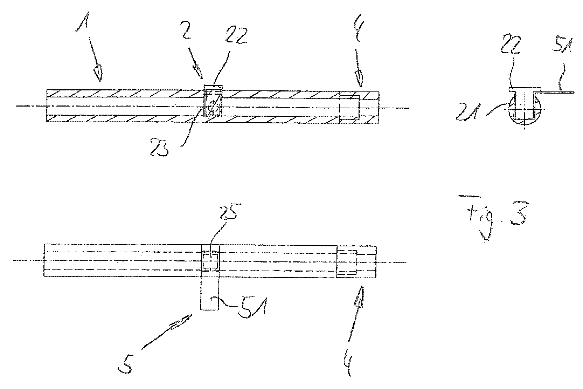

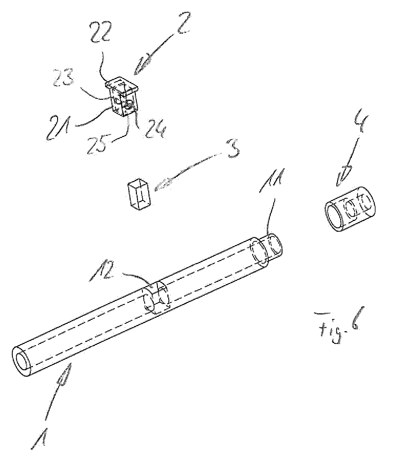

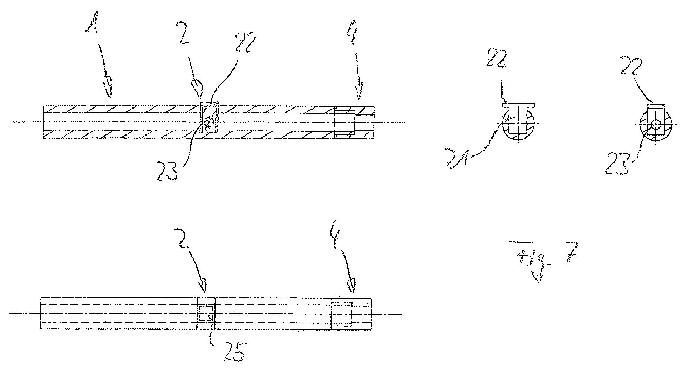

Der Körper 1 ist im Ausführungsbeispiel aus Pappe hergestellt. In Abwandlung des Ausführungsbeispiels können auch andere Materialien, wie beispielsweise Kunststoff oder Metall zur Anwendung kommen. Der Körper 1 ist an seinen beiden Enden offen ausgebildet. An seinem einen Ende ist ein zylindrischer Absatz 11 vorgesehen, der zum Aufstecken des Mundstücks 4 dient. Im mittleren Bereich des Körpers 1 ist zudem eine Aufnahme 12 ausgebildet, in die in den Ausführungsbeispielen nach den Figuren 1 bis 7 ein Depotträger 2 einsetzbar ist; in den übrigen Ausführungsbeispielen nimmt die Aufnahme 12 das Depot 3 unmittelbar auf.

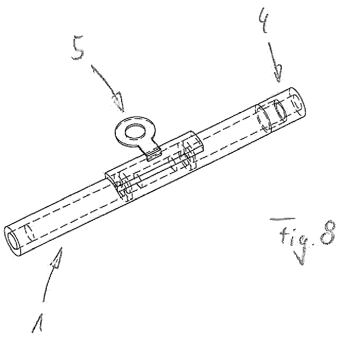

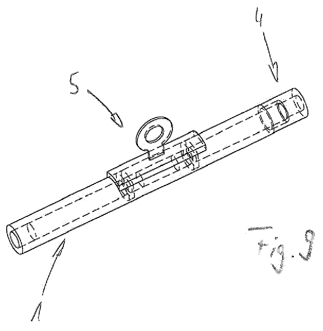

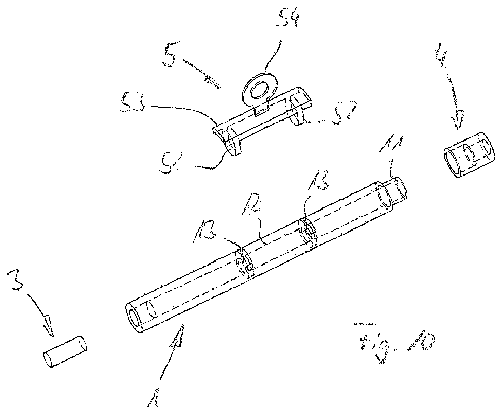

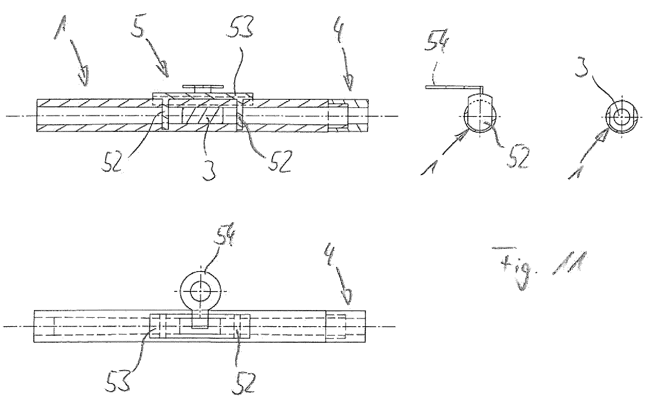

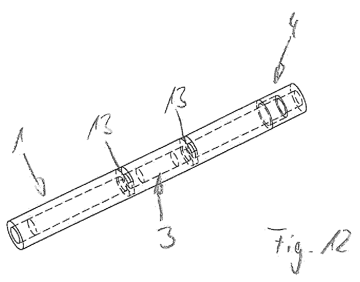

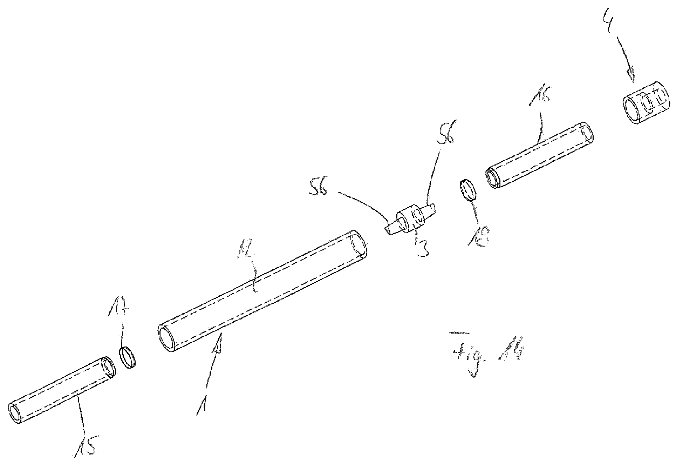

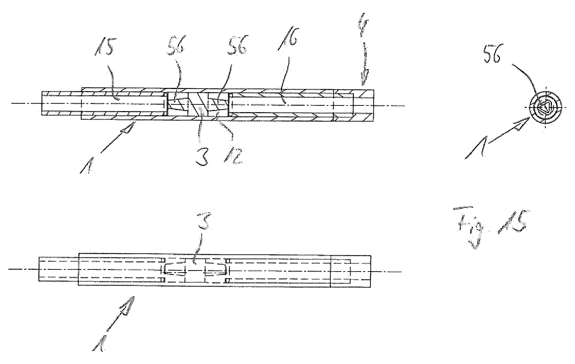

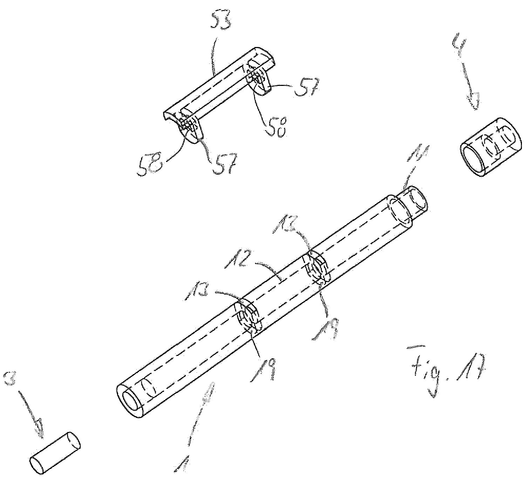

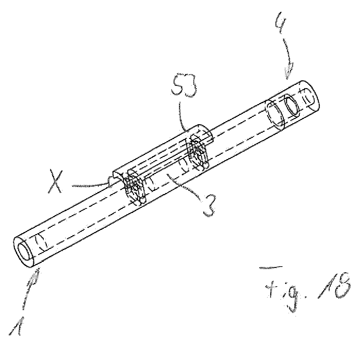

Die Aufnahme 12 kann verschiedene Formen haben, die in Abhängigkeit von der Form des verwendeten Depotträgers 2 bzw. des Depots 3 ausgewählt ist, sodass der Depotträger 2 bzw. das Depot 3 und die Aufnahme 12 problemlos miteinander korrespondieren. Aus diesem Grund weist die Aufnahme 12 im Ausführungsbeispiel nach den Figuren 1 bis 7 eine viereckige Grundform auf; im Ausführungsbeispiel nach den Figuren 8 bis 18 hat die Aufnahme 12 dagegen eine zylindrische Form.

Der Depotträger 2 ist im Ausführungsbeispiel ebenfalls aus Pappe hergestellt. Auch hier ist die Verwendung anderer Materialien, wie beispielsweise Kunststoff oder Metall möglich. Die Verwendung dieser Materialien ermöglicht eine Wiederverwendung des Depotträgers 2 nach Genuss des Inhalts des Depots. Wie bereits vorstehend erwähnt korrespondiert die Form des Depotträgers 2 mit der Form der Aufnahme 12 des Körpers 1. Ebenso korrespondiert die Form des Depots 3 mit der Form des Depotträgers 2. Der Depotträger 2 besteht aus einem quaderförmigen Grundkörper 21 der an seinem einen - oberen - Ende mit einer Griffplatte 22 versehen ist, die auf zwei Seiten den Grundkörper 21 überragt. Der Grundkörper 21 ist hohl ausgebildet und weist an zwei Seiten kreisförmige Öffnungen 23 und 24 auf, die miteinander fluchten. Das Depot 2 weist an seiner Unterseite keinen Boden auf, wodurch eine weitere Öffnung 25 hervorgerufen ist. Durch die Öffnung 25 ist das Depot 3 in den quaderförmigen Grundkörper 21 einsetzbar, welches in dieser Ausführung ebenfalls quaderförmig ist.

Bei dem Depot 3 handelt es sich um einen relativ formstabilen Körper aus gesintertem Material. Alternativ können auch Vliese oder faserige Materialien zur Anwendung kommen. Das Material ist gas- und luftdurchlässig. Das Depot 3 kann verschiedene dreidimensionale Formen aufweisen, beispielsweise die Form eines Quaders, Zylinders, Ellipsoids, einer Kugel oder dergleichen. Es ist mit dem für den jeweiligen Anwendungsfall erforderlichen gasförmigen Medium gefüllt. Im Falle der Benutzung der Vorrichtung als rauchfreie Zigarette ist das Depot 3 mit einem nikotinhaltigen Gas gefüllt.

Die Aktiviereinrichtung 5 ist im Ausführungsbeispiel nach den Figuren 1 bis 3 von einer selbstklebenden Folie 55 gebildet, die die Öffnung 25 auf der Unterseite sowie die beiden mit den Öffnungen 23 und 24 versehenen Seiten umgibt. Nach dem Aufbringen der Folie 55 ist das Depot 3 gasdicht verschlossen. Die Aktiviereinrichtung 5 umfasst in diesem Ausführungsbeispiel eine Abziehvorrichtung in Form einer Lasche 51. Wie den Figuren zu entnehmen ist, ragt die Lasche 51 seitlich über die Griffplatte 22 des Depotträgers 2 hinaus, sodass eine einfache Handhabung gewährleistet ist.

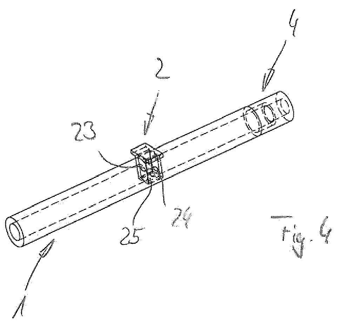

Im nicht erfindungsgemäßen Beispiel nach den Figuren 4 bis 7 ist der Depotträger 2 vergleichbar zu demjenigen in den Figuren 1 bis 3 ausgebildet. Er unterscheidet sich lediglich dadurch, dass er einen im Vergleich etwas vergrößerten Umfang aufweist, sodass er nach dem Einsetzen in die Aufnahme 12 dort unter einem Presssitz positioniert ist, wodurch bereits eine im wesentlichen gasdichte Anordnung geschaffen ist. Zusätzlich kann die Aktiviereinrichtung 5 von einer hauchdünnen Sperrschicht gebildet, die unmittelbar vor dem Einsetzen des Depotträgers 2 in die Aufnahme 12 eingespritzt wird. Nach dem Einsetzen des Depotträgers 2 härtet die Sperrschicht aus, sodass der Bereich gasdicht verschlossen ist. Die Sperrschicht ist dabei vorzugsweise aus Wachs oder Öl gebildet. Wichtig bei der Auswahl des Materials für die Sperrschicht ist ein zuverlässiges Aushärten und Abdichten des Übergangs zwischen Aufnahme 12 und dem Grundkörper 21.

Im nicht erfindungsgemäßen Beispiel nach den Figuren 8 bis 12 ist das Depot 3 nach Art eines Zylinders ausgebildet. In diesem nicht erfindungsgemäßen Beispiel hat die Aufnahme 12 daher einen kreisförmigen Querschnitt. Die Aufnahme 12 ist begrenzt durch Schlitze 13, die rechtwinklig zu seiner Längsmittellinie in dem Körper 1 vorgesehen sind und mit der Aktiviereinrichtung 5 korrespondieren. Hierzu ist die Aktiviereinrichtung 5 mit scheibenförmigen Abdichtungselementen 52 versehen, die in montiertem Zustand in die Schlitze 13 einfahren. Die Abdichtungselemente 52 sind an einem Aktivierungsteil 53 angeordnet, welches eine leicht gewölbte Form aufweist. Das Aktivierungsteil 53 schmiegt sich daher in montiertem Zustand an die Form des Körpers 1 an. An dem Aktivierungsteil 53 ist ein Griffstück 54 angeordnet, welches abklappbar ist. Im nicht erfindungsgemäßen Beispiel ist dies durch das Vorsehen eines Filmscharniers ermöglicht. Die Abdichtung erfolgt mithilfe der zwischen den Schlitzen 13 und den Abdichtungselementen 52 herrschenden Klemmung, die bereits eine ausreichende Abdichtung hervorruft. Zusätzlich besteht jedoch auch hier die Möglichkeit, eine Sperrschicht, unmittelbar vor dem Einsetzen der Abdichtungselemente 52 in die Schlitze 13 einzuspritzen.

Nach dem Aushärten der Sperrschicht ist der Bereich, in dem sich das Depot 3 befindet, gasdicht abgeschlossen. Durch Aufklappen des Griffstücks 54 und Herausziehen der Abdichtungselemente 52 aus den Schlitzen 13 wird die Dichtung aufgehoben. Bei Verwendung der Sperrschicht reißt diese ein, sodass auch dann ein Ansaugen des nikotinhaltigen Gases durch das Mundstück 4 möglich ist.

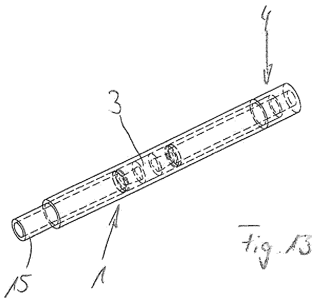

Im nicht erfindungsgemäßen Beispiel nach den Figuren 13 bis 15 ist das Depot 3 mit einem zylindrischen Mittelteil versehen, an dessen beiden stirnseitigen Enden jeweils die Aktivierungseinrichtung 5 in Form von Trennschneiden 56 vorgesehen sind. Das Depot 3 ist in montiertem Zustand etwa im Bereich der Mitte des Körpers 1 angeordnet. In den Körper 1 sind zwei Hülsen 15 und 16 einführbar. Die Hülsen 15 und 16 weisen auf ihren einander zugewandten Seiten jeweils eine Membran 17 und 18 auf, die die Hülsen 15 und 16 einseitig gasdicht verschließen. Die Hülsen 15 und 16 sind ebenso wie der Körper 1 aus Pappe, Kunststoff oder Metall hergestellt.

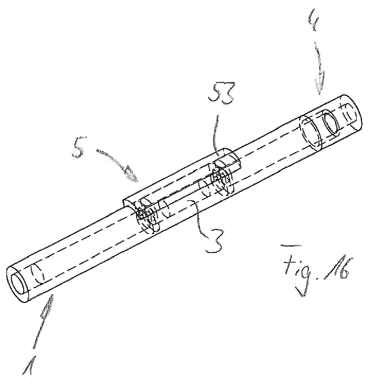

Das nicht erfindungsgemäße Beispiel nach den Figuren 16 bis 18 ist im Wesentlichen vergleichbar zum Beispiel nach den Figuren 8 bis 12 ausgebildet, soweit es das Depot 3, die Aufnahme 12 einschließlich der Schlitze 13 und das Mundstück 4 betrifft. Auch das Aktivierungsteil 53 ist vergleichbar, wobei auf das Griffstück 54 verzichtet ist. Es sind scheibenförmige Abdichtungselemente 57 vorgesehen, die zusätzlich jeweils Durchgangslöcher 58 aufweisen. Darüber hinaus sind in dem Körper 1 auf der den Schlitzen 13 gegenüberliegenden Seite innen Freimachungen 19 ausgebildet. In montiertem Zustand fahren die Abdichtungselemente 57 in die Schlitze 13 ein. In ihrer abgedichteten Position tauchen die Abdichtungselemente 57 lediglich bereichsweise in die Freimachungen 19 ein. In dieser Position ist das Aktivierungsteil 53 um die Höhe "X" beabstandet zum Körper 1 angeordnet. Gleichzeitig befinden sich die Durchgangslöcher 58 noch im Bereich der Schlitze 13, sodass ein Gasdurchtritt verhindert ist. Die Abdichtung erfolgt mithilfe der zwischen den Schlitzen 13 und den Abdichtungselementen 57 herrschenden Klemmung, die bereits eine ausreichende Abdichtung hervorruft. Zusätzlich besteht jedoch auch hier die Möglichkeit, eine Sperrschicht unmittelbar vor dem Einsetzen der Abdichtungselemente 57 in die Schlitze 13 einzuspritzen. Nach dem Aushärten der Sperrschicht ist der Bereich, in dem sich das Depot 3 befindet, gasdicht abgeschlossen.

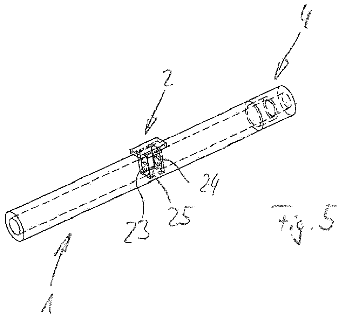

Die Benutzung der erfindungsgemäßen Vorrichtung erfolgt in einfacher Weise: In dem Ausführungsbeispiel nach den Figuren 1 bis 3 ist das quaderförmige Depot 3 von unten in den Grundkörper 21 eingesetzt. Sodann erfolgt im Ausführungsbeispiel nach den Figuren 1 bis 3 das Schließen der Öffnungen 23, 24 und 25 durch Aufbringen der selbstklebenden Folie 55. Durch das Aufbringen der Folie ist das an sich gasdurchlässige Depot gasdicht verschlossen. Der Depotträger 2 wird dann in die Aufnahme 12 eingesetzt. Zur Benutzung der Zigarette wird der Depotträger 2 an der Griffplatte 22 aus der Aufnahme 12 entnommen. Sodann wird die selbstklebende Folie 55 durch Ziehen an der Lasche 51 abgezogen, sodass das nikotinhaltige Gas aus dem Depot 3 austreten kann. Durch Drehen des Depotträgers um 90 Grad und Einsetzen in die Aufnahme 12 besteht die Möglichkeit das Gas aus dem Depot gemeinsam mit Luft über das Mundstück 4 anzusaugen. Zu der Vorgehensweise bei dem Ausführungsbeispiel nach den Figuren 1 bis 3 vergleichbar erfolgt die Vorgehensweise im nicht erfindungsgemäßen Beispiel nach den Figuren 4 bis 7, wobei hier die Abdichtung des Depotträgers 2 in dem Körper 1 durch einen Presssitz bzw. unter Umständen durch Auftragen der Sperrschicht vor dem Einsetzen des Depotträgers 2 in die Aufnahme 12. Nach Aushärten der Sperrschicht ist das Depot 3 gasdicht in dem Körper 1 angeordnet. Durch Herausnehmen des Depotträgers 2 reißt die Sperrschicht ein, sodass das Gas aus dem Depot 3 austreten kann. Durch Drehen des Depotträgers um 90 Grad und anschließendes Einsetzen in die Aufnahme 12 ist ein Ansaugen des gasförmigen Mediums in dem Depot 3 durch das Mundstück 4 möglich, da in dieser Position die Öffnungen 23 und 24 koaxial zum Körper 1 ausgerichtet sind, sodass ein Ansaugen durch das Mundstück 4 möglich ist (vgl. Figuren 4 und 5).

Im nicht erfindungsgemäßen Beispiel nach den Figuren 8 bis 12 erfolgt die Montage zunächst durch Einführen des Depots 3 in den Körper 1, bis dieser seine Position zwischen den Schlitzen 13 im Bereich der Aufnahme 12 eingenommen hat. Um eine positionsgenaue und verrutschsichere Positionierung zu ermöglichen, kann der Körper 1 im Bereich der Aufnahme 12 mit einer Durchmesserverjüngung versehen sein, sodass das Depot 3 unter einer leichten Klemmung in dem Körper 1 gehalten ist. Im Anschluss wird die Aktiviereinrichtung 5 mit den Abdichtungselementen 52 in die Schlitze 13 eingesetzt. Durch den zwischen den Schlitzen 13 und den Abdichtungselementen 52 bestehenden Klemmsitz ist bereits auf diese Weise eine Abdichtung hervorgerufen. Durch Aufbringen einer zusätzlichen Sperrschicht im Bereich der Schlitze 13 und das anschließende Aushärten ist auch hier das an sich gasdurchlässige Depot 3 gasdicht zwischen den Abdichtungselementen 52 angeordnet. Durch Aufklappen des Griffstücks 54 und Ziehen an der Aktiviereinrichtung 5 reißt die optional vorgesehene Sperrschicht im Bereich der Schlitze 13 ein, sodass ein Herausziehen der Abdichtungselemente 52 möglich ist. Das Herausziehen der Abdichtungselemente 52 löst die Abdichtung des Depots 3 auf, wodurch ein Ansaugen der Luft und des beispielsweise nikotinhaltigen Gases durch das Mundstück 4 möglich ist.

Im nicht erfindungsgemäßen Beispiel nach den Figuren 13 bis 15 erfolgt ebenfalls zunächst die Anordnung des Depots 3 im Bereich der Aufnahme 12 des Körpers 1. Auf beiden Seiten des Körpers 1 können im Anschluss die Hülsen 15 und 16 eingeführt werden. Nach Aufsetzen des Mundstücks 4 ragt die Hülse 15 aus dem Körper 1 hervor (Fig. 13). Durch Eindrücken der Hülse 15 tritt die der Hülse zugewandte Trennschneide 56 durch die Membran 17, sodass der gasdichte Verschluss einseitig geöffnet ist. Durch weiteres Einführen der Hülse 15 in den Körper 1 tritt auch die zweite Trennschneide 56 auf der der Hülse 16 zugewandten Seite durch die dort vorgesehene Membran 18, sodass nun ein Ansaugen von Luft und dem im Depot 2 befindlichen Gas möglich ist.

Im nicht erfindungsgemäßen Beispiel nach den Figuren 13 bis 15 erfolgt die Bestückung in zu dem Beispiel nach den Figuren 8 bis 12 vergleichbarer Weise, wobei die Abdichtungselemente 57 jedoch lediglich bereichsweise in den Körper 1 eingeführt werden. Die Abdichtung erfolgt durch den vorhandenen Klemmsitz und kann durch Anbringen einer Sperrschicht unterstützt werden. Durch Drücken auf das Aktivierungsteil 53 fahren die Abdichtungselemente 57 tiefer in die Freimachungen 19 ein. Gleichzeitig treten die Durchgangslöcher 58 aus der Wandung in das Zentrum des Körpers 1 ein. Der Gasdurchtritt wird dadurch freigegeben; ein Ansaugen des Gases aus dem Depot 3 durch das Mundstück 4 ist möglich. Im Falle einer zusätzlichen Abdichtung mittels einer Sperrschicht wird diese beim Drücken auf das Aktivierungsteil 53 zerstört.

## Patentansprüche

1. Vorrichtung zum Inhalieren gasförmiger Medien, insbesondere Nikotin, die aus einem hülsenförmigen Körper (1) besteht, der ein Depot (3) aufnimmt, welches gasdicht verschlossen ist, und die ein in Ansaugrichtung dem Depot (3) nachgeordnetes Mundstück (4) sowie eine Aktivierungseinrichtung (5) zum Öffnen des Depots (3) aufweist, wobei die Vorrichtung nachfüllbar ist, **dadurch gekennzeichnet, dass** die Aktivierungseinrichtung (5) von einer Folie (55) gebildet ist und die Folie (55) selbstklebend ist.

2. Vorrichtung nach Anspruch 1, **dadurch gekennzeichnet, dass** in dem Körper (1) ein Depotträger (2) angeordnet ist, der herausnehmbar ist.

3. Vorrichtung nach Anspruch 1 oder 2, **dadurch gekennzeichnet, dass** in dem Körper (1) eine Aufnahme (12) ausgebildet ist.

4. Vorrichtung nach einem oder mehreren der vorangehenden Ansprüche, **dadurch gekennzeichnet, dass** die Aktivierungseinrichtung (5) eine Abziehvorrichtung umfasst.

5. Vorrichtung nach Anspruch 4 **dadurch gekennzeichnet, dass** die Abziehvorrichtung von einer Lasche (51) gebildet ist.

## Claims

1. Device for inhalation of gaseous media, particularly nicotine, which consists of a sleeve-shaped body (1), which receives a depot (3), which is sealed in a gas-tight manner and which has a mouthpiece (4) arranged downstream of the depot (3) in the intake direction as well as an activation means (5) for opening the depot (3), wherein the device is refillable, **characterised in that** the activation means (5) is formed by a film (55) and the film (55) is self-adhesive.

2. Device according to claim 1, **characterised in that** in the body (1) a depot carrier (2) is arranged, which is removable.

3. Device according to claim 1 or 2, **characterised in that** a receptacle (12) is formed in the body (1).

4. Device according to one or more of the preceding claims, **characterised in that** the activation means (5) comprises a removal device.

5. Device according to claim 4, **characterised in that** the removal device is formed by a tab (51).

## Revendications

1. Dispositif d'inhalation de matières gaseuses, en particulier de nicotine, composé d'un corps (1) en forme de douille recevant une dose (3) obturée étanche aux gaz, et qui présente un embout (4) agencé en aval de la dose (3) dans le sens de l'aspiration ainsi qu'un dispositif d'activation (5) pour ouvrir la dose (3), sachant que le dispositif est rechargeable, **caractérisé en ce que** le dispositif d'activation (5) est formé par un film (55) et que le film (55) est autocollant.

2. Dispositif selon la revendication 1, **caractérisé en ce que** dans le corps (1) est agencé un support (2) amovible de dose.

3. Dispositif selon la revendication 1 ou 2, **caractérisé en ce que** dans le corps (1) est configuré un réceptacle (12).

4. Dispositif selon l'une ou plusieurs des revendications précédentes, **caractérisé en ce que** le dispositif d'activation (5) est un dispositif de pelage.

5. Dispositif selon la revendication 4, **caractérisé en ce que** le dispositif de pelage est formé par une languette (51).
